# EUROPEAN PATENT SPECIFICATION

(11) **EP 2 344 086 B1**
(45) Date of publication and mention of the grant of the patent: **10.04.2013**
(21) Application number: 09786701.4
(22) Date of filing: 24.07.2009
(51) Int. Cl.: A61F 2/38

(54) **ORTHOPEDIC PROSTHESIS**
ORTHOPÄDISCHE PROTHESE
PROTHÈSE ORTHOPÉDIQUE

(30) Priority: 24.07.2008 ZA 200805596
(43) Date of publication of application: 20.07.2011
(73) Proprietor: Oosthuizen, Christiaan Rudolf, 2195 Johannesburg (ZA)
(72) Inventor: Oosthuizen, Christiaan Rudolf, 2195 Johannesburg (ZA)
(74) Representative: MacLean, Martin Robert
(86) International application number: PCT/IB2009/053224
(87) International publication number: WO 2010/010536

(56) References cited:
- US-A- 3 852 830
- US-A- 4 969 907
- US-A- 5 314 482
- US-A1- 2002 068 979
- US-A1- 2002 183 760

## Description

### FIELD OF THE INVENTION

This invention relates to an orthopedic prosthesis. More particularly, this invention relates to an improved resurfacing prosthesis of the knee, and linked thereto an improved surgical approach to knee replacements.

### BACKGROUND OF THE INVENTION

Various types of knee prostheses/prosthetic joints are known in the field of orthopedic surgery, and these are widely used by orthopedic surgeons. In particular, knee prostheses have been developed since the pioneering efforts of Sir John Charnely, and in particular his revolutionary hip replacement techniques introduced during the 1960's.

In order to understand the background to the present invention and the rationale behind knee replacements or revisions, it is necessary to briefly describe the basic anatomy of a knee. A knee joint is formed by the articulation of a femur with a tibia and fibula. The femur has at its substantially cuboid lower extremity two oblong eminences, a lateral and medial condyle. These condyles are separated from each other by a smooth articular depression called a patellar surface. Behind the patellar surface is a deep notch known as an intercondylar fossa.

The medial condyle is longer and, when the femur is held perpendicular to the normal, extends to a lower level as opposed to the lateral condyle. The condyles have a layer of articular cartilage that extends substantially across the anterior, inferior and posterior surfaces of the condyles. The lower and posterior surfaces of these condyles constitute the tibial surfaces for articulation with the corresponding plateaus of the tibia and menisci.

The menisci are two c-shaped fibrocartilage discs disposed between the femur and the tibia. The menisci comprise a lateral meniscus and a medial meniscus, and function to support the femur and tibia, to provide lubrication therebetween (with the assistance of synovial fluid), as well as to cushion for stresses.

Several ligaments function to hold the knee joint in place, and include medial collateral ligaments and lateral collateral ligaments. Also present are the anterior cruciate and the posterior cruciate ligaments.

Problems may arise with knee joints due to various factors, including injury, old age, and genetic predisposition to degenerative diseases. Osteoarthritis can occur after years of normal use of the knee, causing the menisci to crack and wear away. Alignment problems such as bow-legs and so-called "knock knees" may speed up this wear process. A further disease that may lead to problematic knees is rheumatoid arthritis. This is an inflammatory joint disease that destroys the menisci. In both conditions, once the menisci are destroyed or worn away, as the case may be, the femoral condyles will rub against the tibial plateaus, leading initially to pain and swelling and a limited range of movement, and ultimately to a fusing of the joint. Typically, the medial meniscus wears away before the lateral meniscus. This is known as medial compartment arthritis.

The purpose of a knee prosthesis is to provide pain relief, to increase the (presently) restricted range of movement, and to attempt to return a person suffering from a problematic knee to as normal a life as possible. Surgical insertion of the prosthesis is required and is resorted to where no other remedies are available or where such remedies have been exhausted.

Two techniques are available, the first involving an invasive total knee replacement whereby prosthetic devices are surgically attached to both the medial and lateral femoral condyles in aggravated presentations, and a second less-invasive technique known as a partial or unicompartmental knee replacement, whereby a prosthesis is surgically attached to the medial condyle only. In both instances, a corresponding tibial component is surgically attached to the tibial plateau, and a plastic spacer/bearing is inserted therebetween, in order to mimic the functioning of the menisci as these are removed.

A number of prior art prosthesis are known, the most notable of which is the so-called mobile bearing Oxford® Unicompartmental Knee ("*the Oxford knee*"). As described above, this prosthesis consists of a femoral component, a tibial component and a plastic spacer/bearing therebetween. The spacer acts as an articular bearing surface between the tibial and femoral components, respectively. The Oxford knee may have a disadvantage of a limited range of movement and a potential for bearing insert dislocation when the knee is flexed beyond approximately 130°. Furthermore, axial rotation of the knee while the knee is flexed to substantially 90°, or extended to 0°, may result in pronounced spacer/bearing insert overhang, and may further restrict the practical utility of the Oxford knee.

Furthermore, the size and thickness of the femoral component of prior art prostheses, including the Oxford knee, requires a large amount of bone to be removed from the femoral condyle in order to accommodate the prosthesis. This is hardly suitable and because of the limited lifespan of the prior art prostheses (10 - 15 years), a replacement of same may be indicated at that time. Where a substantial amount of bone had been previously removed, a more invasive replacement of the implant may be indicated and a total knee replacement may become necessary, or a revision type of bone augmented replacement.

The challenges in knee replacement prosthetic devices are in summary threefold: to limit the problems of wear, loosening of the prosthesis in use and a post-surgical loss of bone. These represent some of the additional disadvantages associated with the prior art prostheses.

At present, the less invasive uni-compartmental knee replacement is invariably the first step in treating the knee conditions described above. Such prostheses unfortunately usually last for only about 15 years on average, and thereafter a so-called full knee replacement is required. The latter prostheses also last for only about 15 years. Accordingly, one requirement in this field is for knee prostheses to last for longer periods. Another approach to this problem is that, with elderly people living increasingly longer lives, especially in first world countries, an improved technique in such knee procedures could assist such patients in ensuring that such knee prostheses last for an extended period covering the entire remaining lifetime of such patients (which is expected to increase in the future).

US 5314482 discloses a prosthetic femoral component for the knee joint. The prosthesis is suitable for resurfacing chondral deficient surface areas. The femoral component defines a femoral articular bearing surface, and has an elliptical body having an anterior member and a posterior member and an internal femoral attachment surface. The femoral articular bearing surface and the internal femoral attachment surface have a substantially uniform cross-sectional curvature.

### OBJECTS OF THE INVENTION

It is an object of the present invention to provide a new and improved orthopaedic prosthesis that overcomes, at least partially, the disadvantages associated with the prior art, typically in cases of anteromedial arthritis.

It is also an object of the present invention to provide an improved knee prosthesis that involves an inventive step relative to the prior art.

### SUMMARY OF THE INVENTION

According to the present invention, there is provided an orthopedic prosthesis as defined in claim 1.

The elliptical body may be a substantially c-shaped body when viewed from the side. The elliptical body may further be a uniradial body and may provide a free range of movement of approximately 0° to 130° *in situ.*

The cross-sectional thickness of the anterior member may be less than 3 mm thick, and preferably 2 mm thick, at any point along the anterior member. This feature requires less invasive surgery that involves the removal of less bone from the femoral (medial or lateral) condyle.

Also, according to the invention, there may be provided that the posterior member may define a flattened interior surface, thereby defining a posterior fixation point.

The femoral component may have a raised medial edge relative to its lateral edge for movably abutting a corresponding formation provided for on the tibial component, thereby to prevent the femoral component from rotating about an anterior-inferior axis.

The invention further provides that a peg formation may be located on the internal femoral attachment surface, preferably on the anterior member. Furthermore, the peg formation may be oriented to project along the longitudinal axis of the femur. The peg formation may thus define a further fixation point. The peg may be approximately 5 mm in length or longer and provision may be made for the peg to be extendable along its length.

The femoral internal surface may define a suitable surface texture, such as a roughened surface texture for facilitating bony tissue adhesion thereto, while the femoral articular bearing surface may have a highly polished surface for minimizing frictional contact. The roughened surface texture may be fine or rough surface irregularities, The roughened surface texture may further extend onto the surface of the peg formation. The femoral internal surface may further include a mesh, attached to and slightly proud of the femoral internal surface. This mesh may be made from plastics or metal, such as polyethylene or titanium, or a combination thereof.

There is further provided, according to the invention, that the femoral internal surface, inclusive of the peg formation, may be pre-treated or coated with hydroxyapatite, or any suitable growth material, prior to or during surgical placement of the femoral component.

The invention may further provide for the femoral internal surface to have at least one rib formation extending from the flattened interior surface to the peg formation. Preferably the formation is centrally located on the femoral interior surface and extends from the flattened interior surface to just beyond the peg formation. Preferably, the rib may have a height of 1 mm at its highest point and may have a roughened surface and/or mesh.

The femoral component may be made from any suitable material, including but not limited to metal, ceramic, peek, or a blend of these, or a polyethylene, such as ultra high molecular weight polyethylene. Preferably, the femoral component may be made from a metal alloy, such as a surface modified titanium alloy.

The present invention may be used in a revision method of re-surfacing chondral deficient surface areas in the knee joint, wherein the method is minimally invasive, including the step of utilizing and inserting an orthopedic prosthesis including a femoral component, substantially as herein described.

### BRIEF DESCRIPTION OF THE DRAWINGS

The invention will now be described in greater detail, by way of non-limiting example, with reference to the following drawings, in which:
- **FIGURE 1**: shows a schematic perspective view of an improved knee prosthesis *in situ.*
- **FIGURE 2**: shows a schematic cross-sectional front view of the anterior member of femoral component of the knee prosthesis of Figure 1;
- **FIGURE 3**: shows a schematic perspective view of the knee prosthesis of Figure 1, viewed from an opposite (inverted) angle;
- **FIGURE 4**: shows a schematic bottom view of the femoral component of the knee prosthesis of Figure 1;
- **FIGURE 4(A)**: shows a schematic cross-sectional side view of the knee prosthesis of Figure 4 through the line A-A';
- **FIGURE 4(B)**: shows a schematic cross-sectional side view of the knee prosthesis of Figure 4 through the line B-B';
- **FIGURE 4(C)**: shows a schematic cross-sectional side view of the knee prosthesis of Figure 4 through the line C-C';
- **FIGURE 5**: shows a schematic perspective top view of the knee prosthesis of Figure 1;
- **FIGURE 6**: shows a schematic side view of the knee prosthesis of Figure 1;

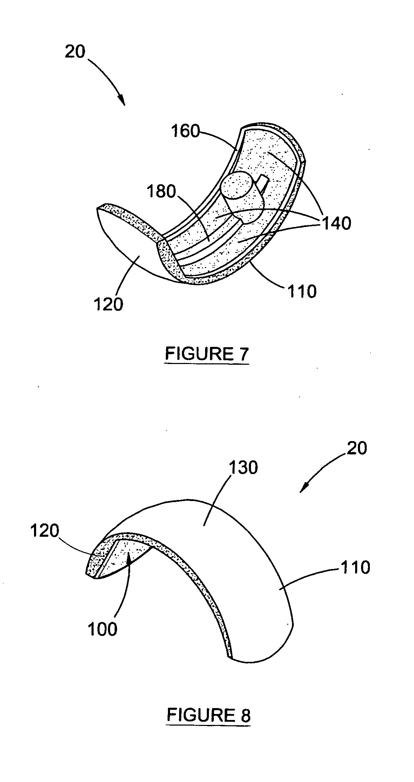

- **FIGURE 7**: shows a schematic perspective view of the knee prosthesis of Figure 1, viewed from an opposite angle; and
- **FIGURE 8**: shows a schematic perspective view of the knee prosthesis of Figure 1, viewed from a different angle wherein the femoral upper bearing surface is illustrated; and
- **FIGURE 9**: shows a schematic top perspective view of the knee prosthesis of Figure 5, including a mesh located on the femoral internal attachment surface.

### DETAILED DESCRIPTION OF THE INVENTION

In the drawings, like numerals refer to like parts, unless otherwise indicated.

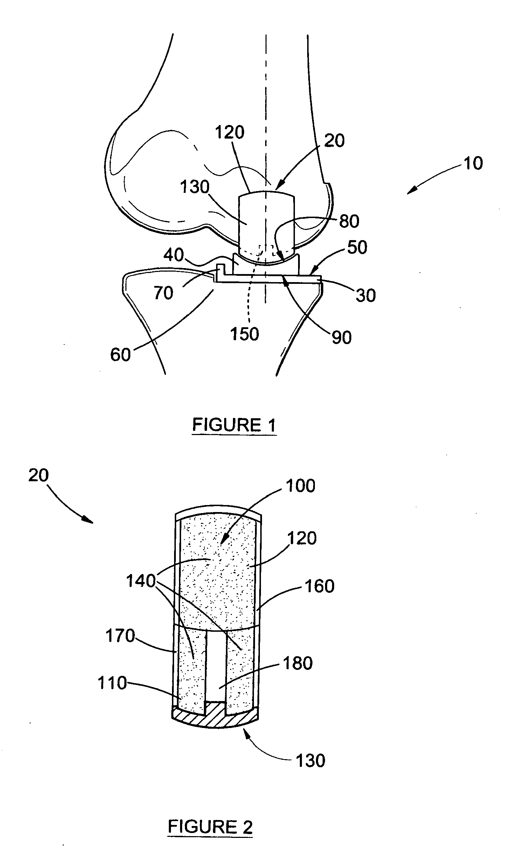

Referring firstly to Figure 1, reference numeral 10 generally refers to an improved orthopedic prosthesis. The orthopedic prosthesis 10 comprises a femoral component generally indicated by reference numeral 20, and a tibial component 30. A spacer bearing 40 is positioned between the femoral component 20 and the tibial component 30.

The tibial component 30 is of the type conventionally utilized in the art and consists of a tibial upper bearing surface 50 in a substantially planar D-shape when viewed in plan view (not shown). The tibial planar upper bearing surface 50 receives the spacer bearing 40 in a floating arrangement. Typically the tibial upper bearing surface 50 is highly polished in order to optimize free movement of the spacer bearing 40 thereon and to reduce friction therebetween. The tibial component 30 further has a lower attachment surface (not shown) for attachment to the tibial plateau 60. A tibial anchor (not shown) in the form of a median fin extending across the tibial lower attachment surface (not shown) relative to the tibial component 30 enhances anchorage of the tibial component 30 to the tibial condyle or tibial plateau 60. These are features that are known in the art. A further feature of the tibial component 30 is an orthogonal lip 70 located on the medial edge of the tibial component 30 for providing a further abutment surface for the spacer bearing 40.

Typically, the spacer bearing 40 is made of a compressed polyethylene manufactured by way of direct compression moulding, as is known in the art. The spacer bearing 40 has an upper bearing surface 80, spherically shaped concavely when viewed in plan view, to accommodate the femoral component 20, as will be described in further detail below. The spacer bearing 40 further has a planar lower bearing surface 90 for movable abutment with the tibial upper bearing surface 50.

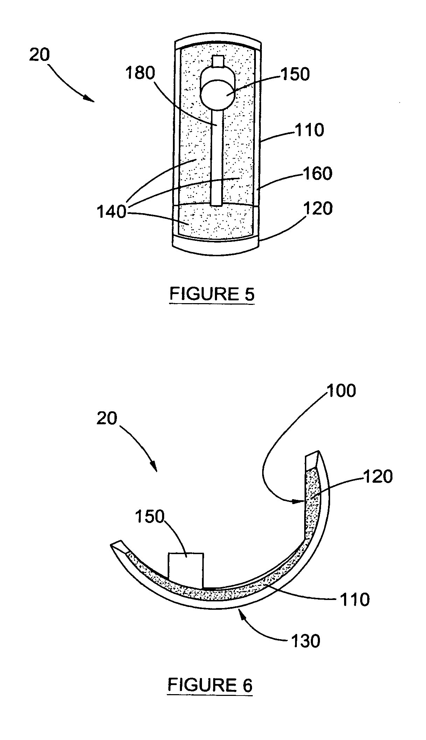

The femoral component 20 has a c-shaped, uni-radial, elliptical body when viewed from the side, as can be seen more clearly in Figure 6. The femoral component 20 has an anterior member 110 and a posterior member 120. The anterior member 110 and the posterior member 120 collectively serve to define the aforementioned elliptical body. The femoral component 20 further has a highly polished femoral articular bearing surface 130 and a femoral internal attachment surface 140. The femoral articular bearing surface 130 engages the upper bearing surface 80 of the spacer bearing 40.

The posterior member 120 defines a flattened posterior (internal) surface 100, which is an extension of the femoral internal attachment surface 140. The elliptical body, and in particular, the femoral internal attachment surface 140 (together with the flattened posterior surface 100) is anatomically shaped to facilitate placement of the femoral component 20 without a significant amount of bone removal.

With reference to Figure 2, the femoral internal attachment surface 140 and the femoral articular bearing surface 130 have a substantially uniform cross-sectional curvature at any point along the length of the anterior member 110. The anterior member 110 has a thickness of 2mm, along its entire length.

The elliptically shaped femoral component 20 thus provides a free range of movement of approximately 0° to 130° *in situ.*

The femoral internal attachment surface 140 has a peg formation 150 located on and extending upwardly from the femoral internal attachment surface 140 of the anterior member 110. The peg formation 150 is oriented to project in a direction along the longitudinal axis of the femur, as can be seen in Figure 1. In the illustrated embodiment, the peg has a basic length of 5 mm. This height is extendable by virtue of a screw and screw threaded bore (not shown) located within the peg formation 150, of the conventional type.

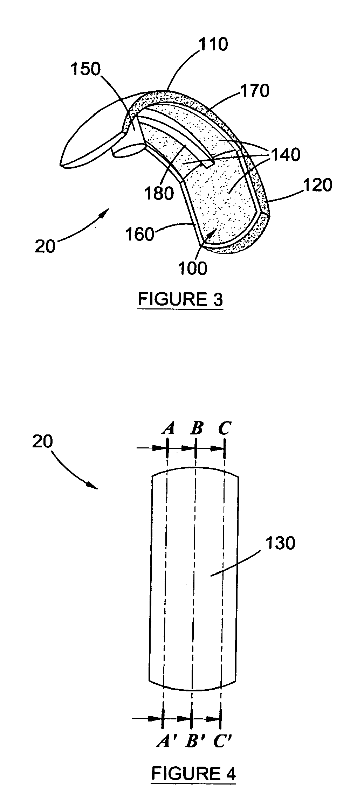

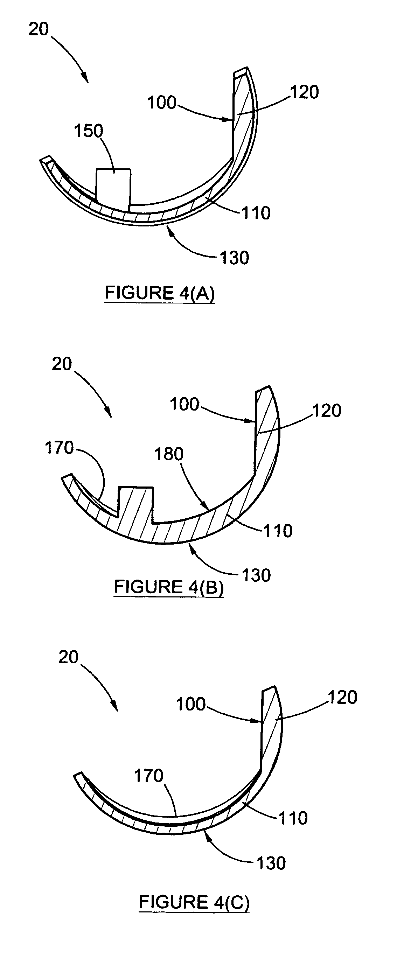

A further line of attachment 160 extends across the substantially outer circumferential edge of the femoral internal attachment surface 140. Furthermore, the femoral component 20 has a medial edge 170 as can be seen most clearly in Figures 4(B) and 4(C). This medial edge 170 movably abuts the orthogonal lip 70 on the tibial component 30.

The femoral internal attachment surface 140 and the peg formation 150 have fine surface irregularities for facilitating bony tissue adhesion thereto, in contrast to the femoral articular bearing surface 130 which has a highly polished surface. The internal attachment surface 140, and the peg formation 150 is coated with hydroxyapatite.

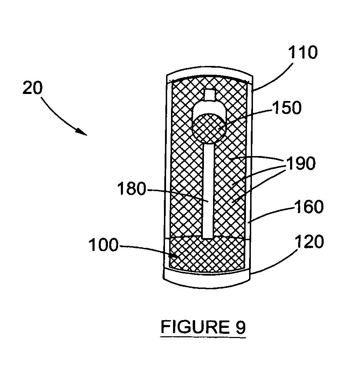

Furthermore, the femoral internal attachment surface 140 has a single, centrally located rib formation 180 extending from the flattened posterior surface 100 to the peg formation 150, and extends just beyond the peg formation 150. This rib formation 180 has a height of 1 mm at its highest point and also has fine surface irregularities. In addition, as can be seen from Figure 9, the femoral internal attachment surface 140 has a metal mesh 190 located on and slightly proud of the femoral internal attachment surface 140.

The femoral component 20 is made from a surface modified titanium alloy.

In use, in a surgical setting, an orthopedic surgeon exposes the medial aspect of the knee for purposes of knee joint revision, as set out herein. While maintaining the integrity of the collateral and cruciate ligaments, and depending on the severity of damage to the surrounding tissue, the medial meniscus is surgically removed. A sufficient amount of bone is also removed from the tibial plateau 60 in order to accommodate the tibial component 30. Further bony incisions are made, as may be necessary, and as determined by the particular tibial component 30 used.

The tibial component 30 is then placed onto the revised tibial plateau 60 and secured thereon using any suitable securing and/or attachment means as is known in the art. Typically, this involves the use of bone cement and may alternatively be cementless, or a combination of these.

The medial femoral condyle is the next subject of revision. Here, bone is removed in order to accommodate the femoral component 20. A bore, aligned with the longitudinal axis of the femur is also introduced in the femoral condyle. The femoral component 20, suitably sized (in small, medium, large or extra large, as the case may be), pre-treated with hydroxyapatite, is then placed onto the area of revision on the femoral condyle, and secured thereto. The aforementioned bore receives the peg formation 150 of the femoral component 20. A suitably sized polyethylene plastic spacer 40 is selected and positioned between the femoral component 20 and the tibial component 30. The incision is surgically closed and allowed to heal. Radiographs may be taken from time to time in order to assess the success of the aforementioned procedure during the healing period.

A person skilled in the art will appreciate the following advantages posed by the present invention, namely, four points of attachment that are of importance and are inherent in the design of the femoral component 20. The most important of these are the increased curvature of the anterior member 110 of the femoral component 20 resulting in an internal surface area increase, which has the advantage of offering a greater cross-sectional line of attachment to bony tissue and hence an increased contact surface when compared to the prior art devices.

A second attachment point relates to the flattened posterior surface 100, which on its own functions to prevent rotation of the femoral component 20 while *in situ.*

A third attachment point, which may work in concert with the second attachment point, relates to the peg formation 150.

A fourth attachment point which enhances the strength of attachment relates to the circumferential line of attachment 160 located on the outer periphery of the internal femoral attachment surface 140 of the femoral component 20.

These attachment points in combination seek to retain the femoral component 20 in site and reduces the probability of aseptic loosening of the femoral component 30, which would result in a failed insert.

A further advantage posed by the femoral component 30 is the reduction in ramp height, without the concomitant removal of a significant amount of bone from the femoral condyle. Ramp height as used in this context means the distance between the femoral condyle and the tibial plateau. This results in less strain being applied to the knee ligaments, especially the cruciate ligaments. Furthermore, by mimicking the natural ramp height of a healthy knee, the right amount of tension will be maintained in the collective knee ligaments, thereby preventing undue pressure being exerted on each respective bearing surface (50, 90, 80 and 130), in the orthopedic prosthesis 10.

With the reduced amount of bony tissue removed from the femoral condyle, the possibility of a further prosthetic replacement, without the resort to a more invasive complete knee replacement, is still viable.

Although certain forms of the invention only have been described herein, it will be understood by any person skilled in the art that other modifications or variations of the invention are possible. For instance, the rib formation 180 located on the femoral internal attachment surface 140 need not be a single rib, and in fact may comprise more than one rib. Further, the peg formation 150 need not be a single peg formation, as more than one peg formation can be used herein. Furthermore, even though the preferred embodiment describes use of the present invention in the case of anteromedial arthritis, the inventor foresees the possibility that the invention may work equally well for lateral femoral condyle resurfacing, or a combination of both medial and lateral resurfacing techniques, as well.

Such modifications and/or variations are therefore to be considered as falling within the scope of the present invention as defined by the claims.

## Claims

1. An orthopedic prosthesis (10) suitable for resurfacing chondral deficient surface areas in knee joints, including a tibial component (30) defining a tibial articular bearing surface (50), a femoral component (20) defining a femoral articular bearing surface (130) and a spacer bearing (40) disposed therebetween, the femoral component further including:
- an elliptical body having an anterior member (110) and a posterior member (120), and an internal femoral attachment surface (140); wherein the interval femoral attachment surface is adapted for bony adhesion thereto and defines an outer circumferential edge located at an outer periphery thereof;
- the femoral articular bearing surface and the internal femoral attachment surface having a substantially uniform cross-sectional curvature at any point along the length of the anterior member, thereby providing an increased radius of the internal femoral attachment surface and an increased cross sectional line of fixation to bony tissue;
- **characterised in that** the internal femoral attachment surface further defined a circumferential line of attachment (160) located on the outer periphery.

2. The orthopedic prosthesis as claimed in claim 1, wherein the elliptical body is a substantially c-shaped body when viewed from the side.

3. The orthopedic prosthesis as claimed in claim 1 or 2, wherein the elliptical body is a uniradial body, and wherein the elliptical body provides a free range of movement of approximately 0° to 130° *in situ.*

4. The orthopedic prosthesis as claimed in any one of the preceding claims, wherein the cross-sectional thickness of the anterior member is less than 3 mm thick, and is preferably 2 mm thick, at any point along the anterior member.

5. The orthopedic prosthesis as claimed in any one of the preceding claims, wherein the posterior member defines a flattened interior surface (100), thereby defining a posterior fixation point.

6. The orthopedic prosthesis as claimed in any one of the preceding claims, wherein a peg formation (150) is located on the internal femoral attachment surface, preferably on the anterior member, and wherein the peg formation defines a further fixation point.

7. The orthopedic prosthesis as claimed in claim 6, wherein the peg formation is oriented to project along the longitudinal axis of the femur.

8. The orthopedic prosthesis as claimed in any one of claims 6, or 7, wherein the peg has a length of 5 mm and is extendable along its length.

9. The orthopedic prosthesis as claimed in any one of the preceding claims, wherein the femoral internal surface defines a suitable surface texture, such as a roughened surface texture for facilitating bony tissue adhesion thereto, while the femoral articular bearing surface has a highly polished surface for minimizing frictional contact.

10. The orthopedic prosthesis as claimed in claim 9, wherein the roughened surface texture is fine or rough surface irregularities, which extends onto the surface of the peg formation.

11. The orthopedic prosthesis as claimed in any one of the preceding claims, wherein the femoral internal surface further includes a mesh (190), attached to and slightly proud of the femoral internal surface, and wherein the mesh surface is consistent with the shape of the femoral internal surface.

12. The orthopedic prosthesis as claimed in claim 11, wherein the mesh is made from plastics or metal, such as polyethylene or titanium, or a combination thereof, and/or wherein the femoral component is made from any suitable material, including but not limited to metal, ceramic, peek, or a blend of these, or a polyethylene, such as ultra high molecular weight polyethylene, preferably a metal alloy, and most preferably a surface modified titanium alloy.

13. The orthopedic prosthesis as claimed in any one of the preceding claims, wherein the femoral internal surface includes the peg formation of claim 6, and is pre-treated or coated with hydroxyapatite, or any suitable growth material, prior to or during surgical placement of the femoral component.

14. The orthopedic prosthesis as claimed in any one of the preceding claims, wherein the femoral internal surface has at least one rib formation (180) extending from the flattened interior surface to the peg formation, the rib formation is preferably centrally located on the femoral interior surface and extends from the flattened interior surface to beyond the peg formation, and wherein the rib formation has a height of 1 mm at its highest point and has a roughened surface and/or mesh..

15. An orthopedic prosthesis including a femoral component as claimed in claim 1, for use in a revision method for re-surfacing chondral deficient surface areas in the knee joint, wherein the method is minimally invasive.

## Patentansprüche

1. Orthopädische Prothese (10), geeignet zur Erneuerung von chondralen defizitären Oberflächen in Kniegelenken, welche eine tibiale Komponente (30), die eine tibialartikuläre Tragfläche (50) definiert, eine femorale Komponente (20), die eine femoral-artikuläre Tragfläche (130) definiert, und ein dazwischen angeordnetes Zwischenraum-Lager (40) einschließt, wobei die femorale Komponente weiterhin einschließt:
- einen elliptischen Körper mit einem vorderen Teil (110) und einem hinteren Teil (120), und einer inneren femoralen Anfügungsfläche (140); wobei die innere femorale Anfügungsfläche zur ossalen Haftung daran angepasst ist und eine äußere umlaufende Kante, angeordnet an einer äußeren Peripherie davon, definiert;
- die femoral-artikuläre Tragfläche und die innere femorale Anfügungsfläche eine im Wesentlichen gleichförmige Querschnitts-Krümmung an einem beliebigen Punkt entlang der Länge des vorderen Teils aufweist, wodurch ein zunehmender Radius der inneren femoralen Anfügungsfläche und eine zunehmende Querschnitts-Linie der Fixierung an ossalem Gewebe bereitgestellt wird;
- **dadurch gekennzeichnet, dass** die innere femorale Anfügungsfläche weiterhin eine umlaufende Befestigungslinie (160), angeordnet an der äußeren Peripherie, definiert.

2. Orthopädische Prothese nach Anspruch 1, wobei der elliptische Körper ein im Wesentlichen c-förmiger Körper ist, wenn er von der Seite betrachtet wird.

3. Orthopädische Prothese nach Ansprüchen 1 oder 2, wobei der elliptische Körper ein uniradialer Körper ist, und wobei der elliptische Körper einen freien Bereich der Bewegung von ungefähr 0° bis 130° in situ bereitstellt.

4. Orthopädische Prothese nach einem der vorangehenden Ansprüche, wobei die Querschnitts-Dicke des vorderen Teils weniger als 3 mm dick ist, und vorzugsweise 2 mm dick ist, an einem beliebigen Punkt entlang des vorderen Teils.

5. Orthopädische Prothese nach einem der vorangehenden Ansprüche, wobei der hintere Teil eine abgeflachte innere Fläche (100) definiert, wodurch ein hinterer Fixierungspunkt definiert wird.

6. Orthopädische Prothese nach einem der vorangehenden Ansprüche, wobei eine Zapfen-Anordnung (150) an der inneren femoralen Anfügungsfläche, vorzugsweise auf dem vorderen Teil, angebracht ist und wobei die Zapfen-Anordnung einen weiteren Fixierungspunkt definiert.

7. Orthopädische Prothese nach Anspruch 6, wobei die Zapfen-Anordnung so ausgerichtet ist, dass sie entlang der Längsachse des Oberschenkelknochens herausragt.

8. Orthopädische Prothese nach einem der Ansprüche 6 oder 7, wobei die Zapfen-Anordnung eine Länge von 5 mm aufweist und entlang ihrer Länge verschiebbar ist.

9. Orthopädische Prothese nach einem der vorangehenden Ansprüche, wobei die innere femorale Fläche eine geeignete Oberflächen-Textur, wie eine aufgeraute Oberflächen-Textur, zum Erleichtern der ossalen Gewebe-Haftung daran, definiert, während die femoral-artikuläre Tragfläche eine stark geglättete Oberfläche zum Minimieren des Reibungs-Kontakts aufweist.

10. Orthopädische Prothese nach Anspruch 9, wobei die aufgeraute Oberflächen-Textur feine oder raue Oberflächen-Unregelmäßigkeiten darstellt, die sich auf der Oberfläche der Zapfen-Anordnung erstrecken.

11. Orthopädische Prothese nach einem der vorangehenden Ansprüche, wobei die innere femorale Fläche weiterhin ein Netz bzw. einen Eingriff (190) einschließt, befestigt an und etwas von der inneren femoralen Fläche herausragend, und wobei die Netz- bzw. Eingriff-Oberfläche mit der Form der inneren femoralen Fläche übereinstimmt.

12. Orthopädische Prothese nach Anspruch 11, wobei das Netz bzw. der Eingriff aus Kunststoff oder Metall, wie Polyethylen oder Titan, oder einer Kombination davon, hergestellt ist und/oder wobei die femorale Komponente aus beliebigem geeignetem Material hergestellt ist, einschließlich, jedoch nicht begrenzt darauf, Metall, Keramik, PEEK oder ein Gemisch von diesen, oder ein Polyethylen, wie Polyethylen mit sehr hohem Molekulargewicht, vorzugsweise eine Metall-Legierung und besonders bevorzugt eine Oberflächen-modifizierte TitanLegierung.

13. Orthopädische Prothese nach einem der vorangehenden Ansprüche, wobei die innere femorale Fläche die Zapfen-Anordnung nach Anspruch 6 einschließt, und vor-behandelt oder beschichtet wird mit Hydroxyapatit oder einem beliebigen geeigneten Wachstumsmaterial, vor oder während des chirurgischen Einbaus der femoralen Komponente.

14. Orthopädische Prothese nach einem der vorangehenden Ansprüche, wobei die innere femorale Fläche mindestens eine Rippen-Anordnung (180) aufweist, die sich von der abgeflachten inneren Fläche zu der Zapfen-Anordnung erstreckt, die Rippen-Anordnung auf der inneren femoralen Fläche vorzugsweise mittig angebracht ist und sich von der abgeflachten inneren Fläche bis hinter die Zapfen-Anordnung erstreckt, und wobei die Rippen-Anordnung an ihrem höchsten Punkt eine Höhe von 1 mm aufweist und eine aufgeraute Oberfläche und/oder ein Netz bzw. einen Eingriff aufweist.

15. Orthopädische Prothese, die eine femorale Komponente nach Anspruch 1 einschließt, zur Verwendung in einem Korrektur-Verfahren zur Erneuerung von chondraldefizitären Oberflächen in dem Kniegelenk, wobei das Verfahren minimal-invasiv ist.

## Revendications

1. Prothèse orthopédique (10) appropriée pour resurfacer des zones superficielles présentant un déficit en cartilage dans les articulations du genou, comprenant un composant tibial (30) définissant une surface d'appui articulaire tibiale (50), un composant fémoral (20) définissant une surface d'appui articulaire fémorale (130) et un appui d'espacement (40) disposé entre elles, le composant fémoral comprenant en outre :
un corps elliptique ayant un élément antérieur (110), un élément postérieur (120), et une surface de fixation fémorale interne (140), dans laquelle la surface de fixation fémorale interne est adaptée pour l'adhésion osseuse à cette dernière et définit un bord circonférentiel externe positionné au niveau de sa périphérie externe ;
la surface d'appui articulaire fémorale et la surface de fixation fémorale interne ayant une courbure transversale sensiblement uniforme tout le long de l'élément antérieur, fournissant ainsi un rayon accru de la surface de fixation fémorale interne et une ligne transversale accrue de fixation au tissu osseux ;
**caractérisée en ce que** la surface de fixation fémorale interne définit en outre une ligne circonférentielle de fixation (160) positionnée sur la périphérie externe.

2. Prothèse orthopédique selon la revendication 1, dans laquelle le corps elliptique est un corps sensiblement en forme de C, lorsqu'il est observé depuis le côté.

3. Prothèse orthopédique selon la revendication 1 ou 2, dans laquelle le corps elliptique est un corps uniradial, et dans laquelle le corps elliptique fournit une plage libre de mouvement d'approximativement 0° à 130° in situ.

4. Prothèse orthopédique selon l'une quelconque des revendications précédentes, dans laquelle l'épaisseur transversale de l'élément antérieur est inférieure à 3 mm et est de préférence de 2 mm, à n'importe quel point le long de l'élément antérieur.

5. Prothèse orthopédique selon l'une quelconque des revendications précédentes, dans laquelle l'élément postérieur définit une surface intérieure aplatie (100), définissant ainsi un point de fixation postérieur.

6. Prothèse orthopédique selon l'une quelconque des revendications précédentes, dans laquelle une formation de cheville (150) est positionnée sur la surface de fixation fémorale interne, de préférence sur l'élément antérieur, et dans laquelle la formation de cheville définit un autre point de fixation.

7. Prothèse orthopédique selon la revendication 6, dans laquelle la formation de cheville est orientée pour faire saillie le long de l'axe longitudinal du fémur.

8. Prothèse orthopédique selon l'une quelconque des revendications 6 ou 7, dans laquelle la cheville a une longueur de 5 mm et est extensible le long de sa longueur.

9. Prothèse orthopédique selon l'une quelconque des revendications précédentes, dans laquelle la surface interne fémorale définit une texture de surface appropriée, telle qu'une texture de surface rugueuse pour faciliter l'adhésion du tissu osseux sur cette dernière, alors que la surface d'appui articulaire fémorale a une surface très polie pour minimiser le contact de friction.

10. Prothèse orthopédique selon la revendication 9, dans laquelle la texture de surface rugueuse est une irrégularité de surface fine ou rugueuse qui s'étend sur la surface de la formation de cheville.

11. Prothèse orthopédique selon l'une quelconque des revendications précédentes, dans laquelle la surface interne fémorale comprend en outre une maille (190) fixée sur et légèrement en saillie de la surface interne fémorale, et dans laquelle la surface de maille est compatible avec la forme de la surface interne fémorale.

12. Prothèse orthopédique selon la revendication 11, dans laquelle la maille est réalisée à partir de plastique ou de métal, tel que le polyéthylène ou le titane, ou leur combinaison, et/ou dans laquelle le composant fémoral est réalisé à partir de n'importe quel matériau approprié comprenant sans y être limité, le métal, la céramique, le polyétheréthercétone ou un mélange de ces derniers, ou un polyéthylène, tel que le polyéthylène à poids moléculaire ultra-élevé, de préférence un alliage de métal, et de manière préférée entre toutes un alliage de titane modifié en surface.

13. Prothèse orthopédique selon l'une quelconque des revendications précédentes, dans laquelle la surface interne fémorale comprend la formation de cheville selon la revendication 6, et est prétraitée ou recouverte avec de l'hydroxyapatite ou n'importe quel matériau de croissance approprié, avant ou pendant la mise en place chirurgicale du composant fémoral.

14. Prothèse orthopédique selon l'une quelconque des revendications précédentes, dans laquelle la surface interne fémorale a au moins une formation de nervure (180) s'étendant à partir de la surface intérieure aplatie jusqu'à la formation de cheville, la formation de nervure est de préférence positionnée de manière centrale sur la surface intérieure fémorale et s'étend à partir de la surface intérieure aplatie jusqu'au-delà de la formation de cheville, et dans laquelle la formation de nervure a une hauteur de 1 mm à son point le plus haut et a une surface rugueuse et/ou maille.

15. Prothèse orthopédique comprenant un composant fémoral selon la revendication 1, destiné à être utilisé dans un procédé de révision pour resurfacer des zones superficielles présentant un déficit en cartilage dans l'articulation du genou, dans laquelle le procédé est minimalement invasif.
